# EUROPEAN PATENT SPECIFICATION

(11) **EP 3 634 281 B1**
(45) Date of publication and mention of the grant of the patent: **07.08.2024**
(21) Application number: 18734405.6
(22) Date of filing: 06.06.2018
(51) Int. Cl.: A61B 17/84, A61B 17/68, A61B 17/04

(54) **DEVICES FOR TISSUE REPAIR**
VORRICHTUNGEN ZUR GEWEBEREPARATUR
DISPOSITIFS POUR LA RÉPARATION DE TISSUS

(30) Priority: 09.06.2017 US 201762517515 P
(43) Date of publication of application: 15.04.2020
(73) Proprietor: Smith & Nephew, Inc., Memphis, TN 38116 (US)
(72) Inventor: BOILEAU, Pascal, Memphis, Tennessee 38116 (US); BETTENGA, Mason, Memphis, Tennessee 38116 (US)
(74) Representative: Appleyard Lees IP LLP
(86) International application number: PCT/US2018/036286
(87) International publication number: WO 2018/226852

(56) References cited:
- WO-A1-2010/019384
- WO-A1-2017/062300
- WO-A1-2017/218167
- US-A1- 2009 228 049
- US-A1- 2010 211 075

## Description

### FIELD

This disclosure relates to devices for tissue repair. In particular, this disclosure relates to devices for obtaining fusion between two pieces of bone, for example, between a bone graft and a glenoid during a glenohumeral instability repair.

### BACKGROUND

The shoulder joint, also referred to as the glenohumeral joint, is the joint between the glenoid cavity (a part of the scapula) and the head of the humerus (upper arm bone). The glenoid cavity is shallow, covering only about a third of the head humeral head. As a result, the glenoid cavity provides relatively little bony constraint upon motion of the humerus and the glenohumeral joint exhibits the widest range of motion of all joints in the human body. While the glenohumeral joint is also constrained by soft tissue (e.g., cartilage attached to the rim of the glenoid cavity, tendons, etc.), soft tissue in general cannot provide the same degree of constraint as bone. Accordingly, it is relatively easy to force the humerus from its normal anatomical position with respect to the glenoid socket, that is, to dislocate the shoulder. While not life threatening, a dislocated shoulder can cause pain and immobilization of the joint, impacting a patient's lifestyle.

In the case of severe bone loss caused by shoulder instability and/or dislocation, a surgeon may perform a "Latarjet procedure" to make the repair. In a Latarjet procedure, a surgeon attempts to restore bone mass to the glenoid cavity by securing a bone graft to the surface of the glenoid suffering bone loss. The bone graft may or may not be attached to soft tissue. When successful, the bone graft acts as a scaffold, allowing the glenoid bone to grow into the bone graft and restore the lost glenoid bone mass.

During the Latarjet procedure, fixation devices, such as solid screws, may be used to provide compression when securing the graft to the bone. Alternatively, if suspension fixation is desired, the fixation devices may be in the form of a flat button that is positioned on a surface of the bone graft and/or bone and is tensioned in place by a suture. However, with suspension fixation, such flat button fixation devices provide very little shear stability across the fracture line compared to solid screws. US2010211075A1 relates to devices and methods for fracture fixation, and more particularly to holding bone fragments together to permit healing. WO2017/062300 relates to an adjustable apparatus that at least assists in maintaining tension on a one or more bones during joint repair and/or healing. WO2017/218167 relates to a fixation device that uses suspension fixation while incorporating a long post attached to a flat button. WO2010/019384 relates to a bone compression device for fixing first and second bone fragments together. US2009/228049 relates to connecting cannulated bone screws.

### SUMMARY

The invention is defined in independent claim1. Certain optional features are defined in the dependent claims. The methods described herein do not form part of the invention. Described herein is a fixation device that uses suspension fixation while incorporating a long post attached to a flat button. The long post is solid and provides a shear stability greater than or equal to a screw. When two or more fixation devices are used, the fixation devices also provide rotational stability of the bone graft with respect to the underlying bone. The fixation devices of this disclosure can also be passed through anatomical structures arthroscopically much easier than standard screws and may allow the entire repair to be done using a single skin portal, rather than multiple portals.

The tissue fixation construct of this disclosure include a first fastener having a generally flat, circular first body, and a generally cylindrical, solid first post fixedly coupled to a center of the first body and extending perpendicular to the first body. The first post has a threaded distal end and a non-threaded proximal end. A length of the first post is selected to extend through a bone graft and a portion of bone. The tissue fixation construct also includes a second fastener having a generally flat, circular second body and a generally cylindrical, solid second post fixedly coupled to a center of the second body and extending perpendicular to the second body. The second fastener also has a cannulation extending through the second body and the second post. At least a portion of the cannulation is threaded to engage the threaded distal end of the first post of the first fastener. A length of flexible material coupled to the distal end of the first post. The flexible cable (60) is configured to be pulled through the cannulation (80) of the second fastener (70). In further examples, a length of the first post is about 35 mm. The tissue fixation construct also includes. A distal end of the length of flexible material includes a loop.

The fixation device of this disclosure is described as used during a tissue graft fixation in a glenohumeral instability repair. However, it is important to note that the fixation device could be used anywhere in the body to obtain the fusion between two pieces of bone. For example, the fixation device could also be used to repair fracture in the glenoid, a malleolar fracture in the ankle, or an oblique fracture of the femur. It is contemplated that the fixation device of this disclosure could also be coupled with plate fixation.

In examples, the surgical fastener of this disclosure includes a generally flat, circular body having a first surface and a second surface opposite the first surface and a generally cylindrical, solid post. The post has a first end, a second end, and a longitudinal axis extending between the first and second ends, the longitudinal axis being perpendicular to the second surface of the body. The first end of the post is fixedly coupled to a center of the second surface. A length of the post is selected to extend through a bone graft and a portion of bone.

In further examples, the body also includes a first through hole extending from the first surface to the second surface, the first through hole being located at the center of the second surface. In this example, the post further has a second through hole extending along the longitudinal axis from the first end to the second end, the second through hole in communication with the first through hole. The first and second through holes are configured for passage of a suture. In other examples, the body further includes a pair of first through holes extending from the first surface to the second surface, the pair of first through holes being symmetrically offset from the center of the second surface. In this example, the post further has an eyelet at the second end of the post transverse to the longitudinal axis. The first pair of through holes and the eyelet are configured for the passage of a suture. In yet other examples, the length of the post is between about 20 mm and about 25 mm. A radius across the first surface of the body is about 7 mm. A diameter of the post is about 2.8 mm.

In further examples, the body also includes a first through hole extending from a first surface to an opposite second surface, the first through hole being located at the center of the body. In this example, the post further includes a second through hole extending along the longitudinal axis from the first end to the second end, the second through hole in communication with the first through hole. The first and second through holes are configured for passage of a suture. In other examples, the body further includes a pair of first through holes extending from a first surface to an opposite second surface, the pair of first through holes being symmetrically offset from the center of the body. In this example, the post further has an eyelet at the second end of the post transverse to the longitudinal axis. The first pair of through holes and the eyelet are configured for the passage of a suture. In yet further examples, a length of the post of the one of the first or second fasteners is between about 20 mm and about 25 mm. A diameter of the post of the one of the first or second fasteners is about 2.8 mm. A diameter of the at least one axially aligned passage is about 2.8 mm. The method further includes tying a surgical knot in the suture.

Examples of the tissue fixation construct of this disclosure include a first fastener and a second fastener coupled to the first fastener by a suture such that a distance between the first fastener and the second fastener can be adjusted by pulling on ends of the suture. One of the first fastener or the second fastener has a generally flat, circular body and a generally cylindrical, solid post having a first end, a second end, and a longitudinal axis extending between the first and second ends. The longitudinal axis is perpendicular to the body, and the first end of the post is fixedly coupled to a center of the body. A length of the post is selected to extend through a bone graft and a portion of bone.

In further examples, the body also includes a first through hole extending from a first surface to an opposite second surface, the first through hole being located at the center of the body. In this example, the post further has a second through hole extending along the longitudinal axis from the first end to the second end, the second through hole in communication with the first through hole. The first and second through holes are configured for passage of a suture. In other examples, the body further includes a pair of first through holes extending from a first surface to an opposite second surface, the pair of first through holes being symmetrically offset from the center of the body. In this example, the post further has an eyelet at the second end of the post transverse to the longitudinal axis. The first pair of through holes and the eyelet configured are for the passage of a suture. In yet further examples, a length of the post of the one of the first or second fasteners is between about 20 mm and about 25 mm. The other of the first or second fasteners includes a suture slidably coupled to the other of the first or second fasteners, the suture in the form of a suture loop. In examples, the other of the first or second fasteners comprises a rectangular body.

These and other features and advantages will be apparent from a reading of the following detailed description and a review of the associated drawings. It is to be understood that both the foregoing general description and the following detailed description are explanatory only and are not restrictive of aspects as claimed.

### BRIEF DESCRIPTION OF THE DRAWINGS

The disclosure will be more fully understood by reference to the detailed description, in conjunction with the following figures, wherein:
FIGS. 1A and 1B are illustrations of an example of the fixation device of this disclosure;
FIGS. 2A and 2B are illustrations of another example of the fixation device of this disclosure;
FIGS. 3A-C illustrate a method of tissue repair using the fixation device of FIGS. 1A and 1B;
FIGS. 4A-C illustrate a method of tissue repair using the fixation device of FIGS. 2A and 2B;
FIGS. 5A-E illustrate examples of an ancillary fastener to be used in the methods of tissue repair shown in FIGS. 3A-C and FIGS. 4A-C;
FIG. 6A illustrates another example of the fixation device of this disclosure;
FIG. 6B illustrates another example of an ancillary fastener to be used with the fixation device of FIG. 6A during a tissue repair;
FIGS. 6C and 6D illustrate a method of tissue repair using the fixation device and ancillary fastener of FIGS. 6A and 6B; and
FIGS. 7A and 7B illustrate a method of tissue repair using the fixation device of FIGS. 1A and 1B and a rectangular ancillary fastener.

### DETAILED DESCRIPTION

In the description that follows, like components have been given the same reference numerals, regardless of whether they are shown in different examples. To illustrate example(s) in a clear and concise manner, the drawings may not necessarily be to scale and certain features may be shown in somewhat schematic form. Features that are described and/or illustrated with respect to one example may be used in the same way or in a similar way in one or more other examples and/or in combination with or instead of the features of the other examples.

Comprise, include, and/or plural forms of each are open ended and include the listed parts and can include additional parts that are not listed. And/or is open ended and includes one or more of the listed parts and combinations of the listed parts.

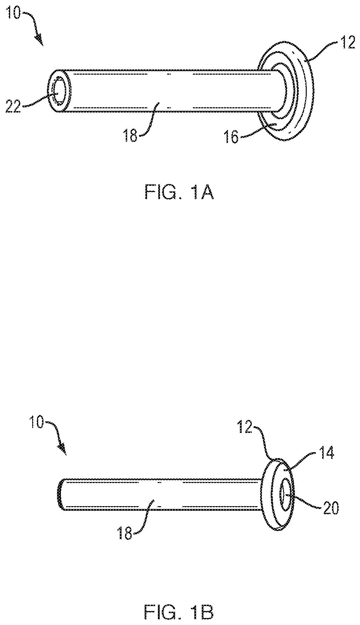

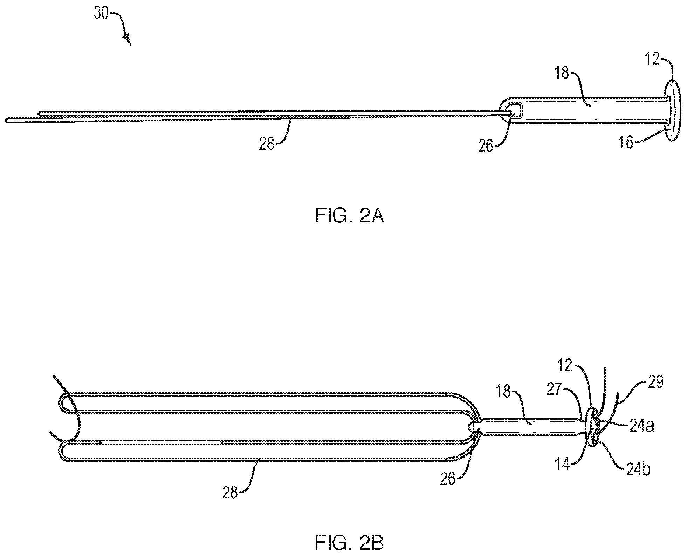

Two examples of a fixation device 10, 30, according to the present disclosure, are illustrated in FIGS. 1A-B and 2A-B. In both examples, the fixation devices 10, 30 include a generally circular body 12 having a first surface 14, which may be flat or convex, for facing away from bone, and an opposite concave (or bowl-shaped) second surface 16, for facing bone. All edges of the body 12 are rounded to avoid chafing of a suture or graft. A radius across the first surface 14 of the body 12 may be about 7 mm. However, the radius may also be varied to accommodate a patient or procedure. The fixation device 10, 30 further includes a generally cylindrical, solid post 18 fixedly coupled to a center of the body 12 and extending perpendicular to the body 12. A length of the post 18 is selected to extend through both of a bone graft and a portion of bone, such as a glenoid bone. For example, the thickness of a bone graft may be about 5 mm to about 11 mm, while a length of the post 18 may be about 20 mm to about 25 mm, although the post 18 could be longer in some applications. Advantageously, as described in further detail below, the post 18 provides greater or equal shear support to the fixation device 10, 30 across the fracture line of the bone as compared to surgical screws. Furthermore, when two or more fixation devices 10, 30 are used, the fixation devices 10, 30 additionally provide rotational stability to the bone graft with respect to the underlying bone.

The body 12 and the post 18 can each be formed of surgical quality stainless steel. Other biocompatible materials are acceptable, such as a Delrin polymer available from Du Pont or a bioabsorbable material such as polylactic acid, polyglycolic acid disclosed in U.S. Pat. No. 3,739,773 (Schmitt et al.), or copolymers disclosed in U.S. Pat. Nos. 4,300,565 (Rosensaft et al.) and 4,429,080 (Casey et al.). A combination of absorbable and non-absorbable materials to form a partially absorbable fixation device can also be utilized. A polymer such as polylactic acid may be preferred for its slower absorption rate and therefore longer retention of structural integrity.

In the example of the fixation device 10 shown in FIGS. 1A-B, the body 12 further comprises a first through hole 20 extending from the first surface 14 to the second surface 16 of the body 12, the first through hole 20 being located at the center of the body 12. In this example, the post 18 also includes a second through hole 22 extending along the longitudinal axis of the post 18, the second through hole 22 being in communication with the first through hole 20. The first and second through holes 20, 22 are each configured for passage of a suture, as further described below. Diameters of the first and second through holes 20, 22 are variable and are selected based on the size of the suture to be used.

In the example of the fixation device 30 shown in FIGS. 2A-B, the body 12 further comprises pair of first through holes 24a, 24b extending from the first surface 14 to the second surface 16 of the body 12. The pair of first through holes 24a, 24b are symmetrically offset from the center of the body 12 and are not obstructed by the post 18. The pair of first through holes 24a, 24b are configured for the passage of a suture, such as a lead or pull suture 29. In this example, the post 18 also includes an eyelet 26 extending through the distal end of the post 18 for passage of a suture. A tension suture 28 may be slidably coupled to the eyelet 26. The form of the tension suture 28 may be varied, in certain examples. For example, the tension suture 28 may be formed in a suture loop or bundle. In further examples, the tension suture 28 may be formed from a high-strength polyethylene or may be formed from metallic wire. In some examples, the post 18 also includes an eyelet 27 extending through the proximal end of the post 18 for passage of an additional suture, such as the pull suture 29. Sizes of the eyelets 26, 27 are variable, and are selected based on the size of the suture used.

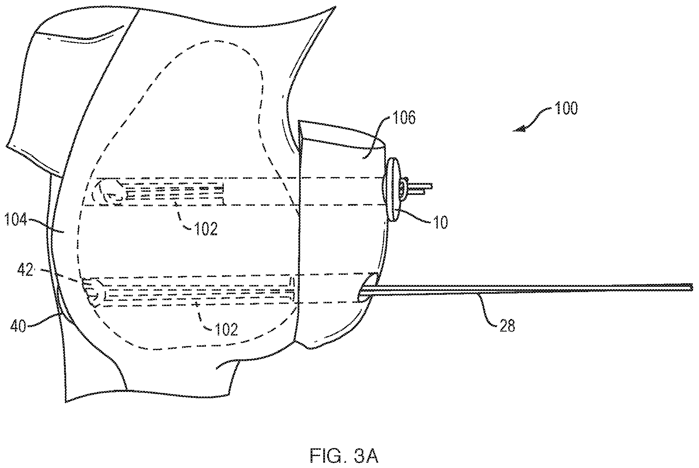

FIGS. 3A-C illustrate a method of tissue repair using fixation device 10 as described above. Referring to FIG. 3A, in which a shoulder 100 of a patient is illustrated, axially aligned passages 102, which may be two passages placed about 10 mm apart, are initially drilled through both of the patient's glenoid 104 and a graft, such as a bone graft 106. In examples, the bone graft 106 may be a coracoid bone graft, an iliac crest bone graft or an allograft. After the passages 102 are formed, the patient's shoulder 100 is prepared for insertion of the fixation device 10. Non-limiting examples of methods for preparing a patient's shoulder are described in U.S. Patent Publication No. 2014-0277185 (Boileau et al.), incorporated herein by reference. An ancillary fastener 40, such as a round button, may be pre-attached to a tension suture 28. Non-limiting examples of ancillary fasteners 40 are described in U.S. Patent Publication No. 2012/0310279 (Sikora et al.), U.S. Patent Publication No. 2014-0277185 (Boileau et al.), and in the Endobutton family of products (manufactured by Smith & Nephew, Inc., Andover, MA, USA). The tension suture 28 is slidably passed through an opening 42 in the ancillary fastener 40, or otherwise coupled to the ancillary fastener 40, such that the distance between the ancillary fastener 40 and the fixation device 10 can be adjusted by pulling on the ends of the tension suture 28, as further described below. In FIG. 3A, the ends of the tension suture 28 are pulled, for example by shuttling suture (not shown), through the passages 102 in the patient's glenoid 104 and bone graft 106, until the ancillary fastener 40 is positioned below the glenoid 104 and the ends of the tension suture 28 extend from passages 102 in the bone graft 106.

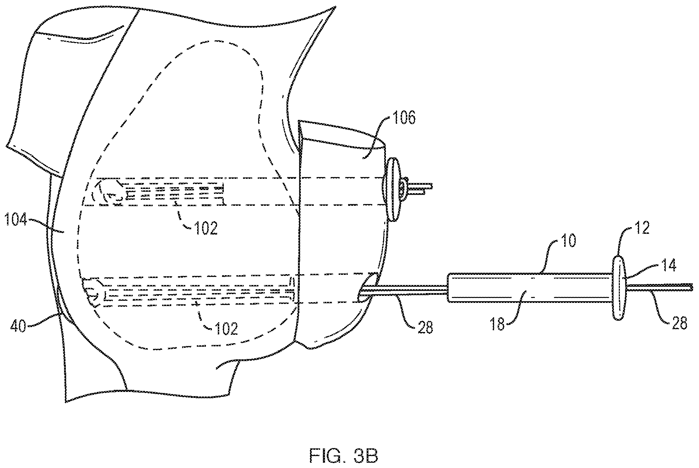

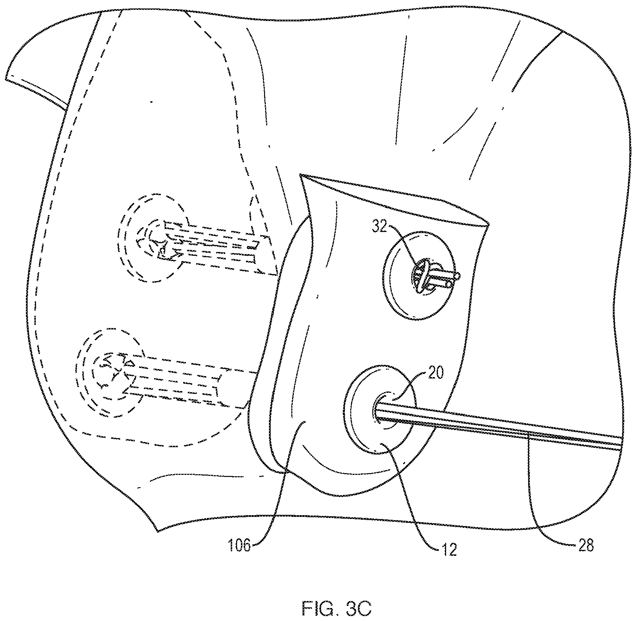

As shown in FIG. 3B, with the ancillary fastener 40 positioned below the glenoid 104, the ends of the tension suture 28 are passed through the post 18 of fixation device 10 such that they exit the first surface 14 of the body 12. In one example, the diameter of the post 18 is selected to be substantially equal to the diameter of the passages 102, which may be about 2.8 mm. Since the passages 102 are formed through relatively soft, deformable bone, as the ends of the tension suture 28 are pulled, the fixation device 10 is reduced toward the bone graft 106 such that the post 18 is inserted into the passages 102, forming a slip fit with the passages 102. Once fully-inserted, the post 18 extends completely through the passages 102 of the bone graft 106 and at least partially through the passages 102 of the glenoid 104. As shown in FIG. 3C, once the bone graft 106 is in the preferred position, a surgical knot 32 is tied in the tension suture 28 above the first through hole 20 of the body 12, fixing the bone graft 106 into place. The ends of the tension suture 28 may then be trimmed.

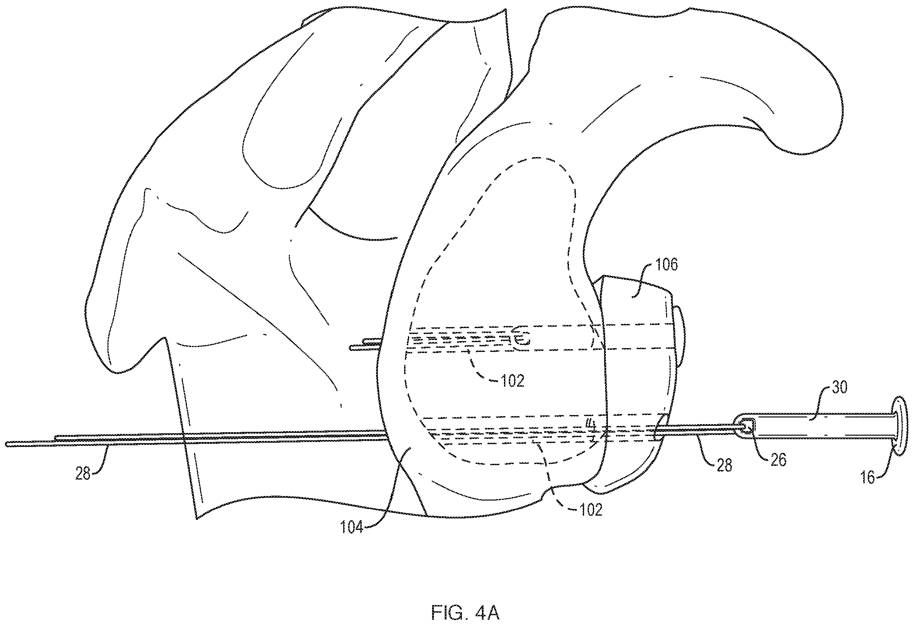

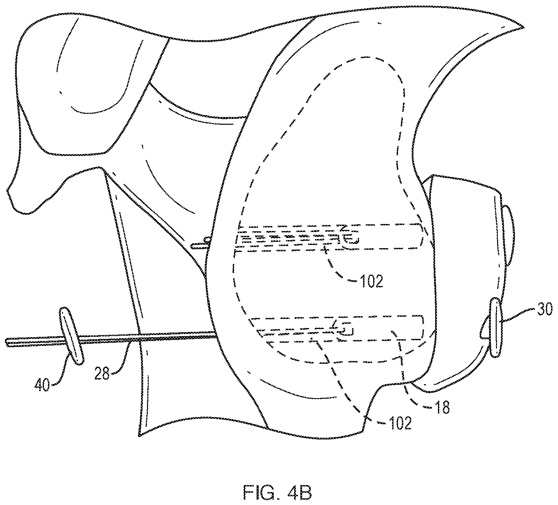

FIGS. 4A-C illustrate a method of tissue repair using fixation device 30 as described above. Referring to FIG. 4A, axially aligned passages 102 are drilled through the patient's glenoid 104 and a bone graft 106. A tension suture 28 is slidably coupled to the eyelet 26 of the fixation device 30 such that the distance between the fixation device 30 and an ancillary fastener 40 (FIG. 4B) can be adjusted by pulling on the ends of the tension suture 28. In FIG. 4A, the ends of the tension suture 28 are pulled through the passages 102 in the bone graft 106 and the patient's glenoid 104, until the second surface 16 of the fixation device 30 is positioned above the surface of the bone graft 106 and the ends of the tension suture 28 extend from passages 102 below the glenoid 104.

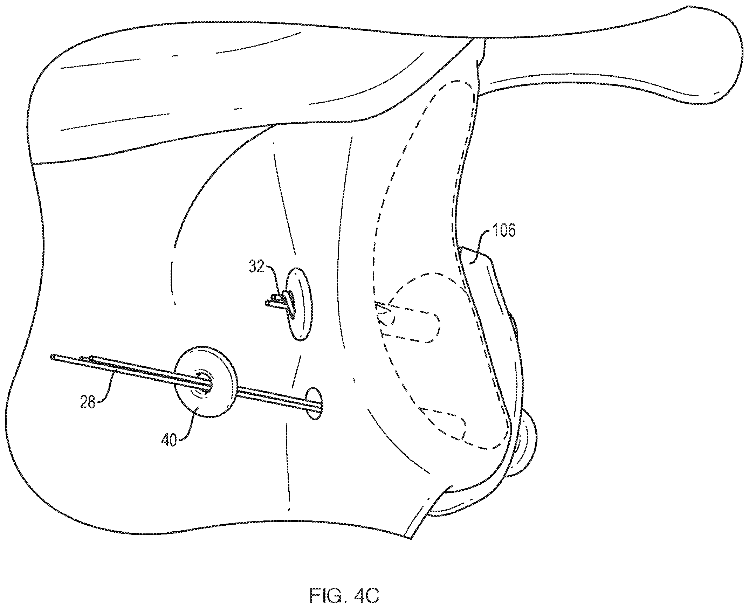

As shown in FIG. 4B, as the ends of the tension suture 28 are pulled, the fixation device 30 is reduced toward the bone graft 106 such that the post 18 is inserted into the passages 102. Once fully-inserted, the post 18 extends completely through the passages 102 of the bone graft 106 and at least partially through the passages 102 of the glenoid 104. The tension suture 28 is then coupled to an ancillary fastener 40. As shown in FIG. 4C, once the bone graft 106 is in the preferred position, a surgical knot 32 is tied in the tension suture 28 below the ancillary fastener 40, fixing the bone graft 106 into place. The ends of the tension suture 28 may then be trimmed.

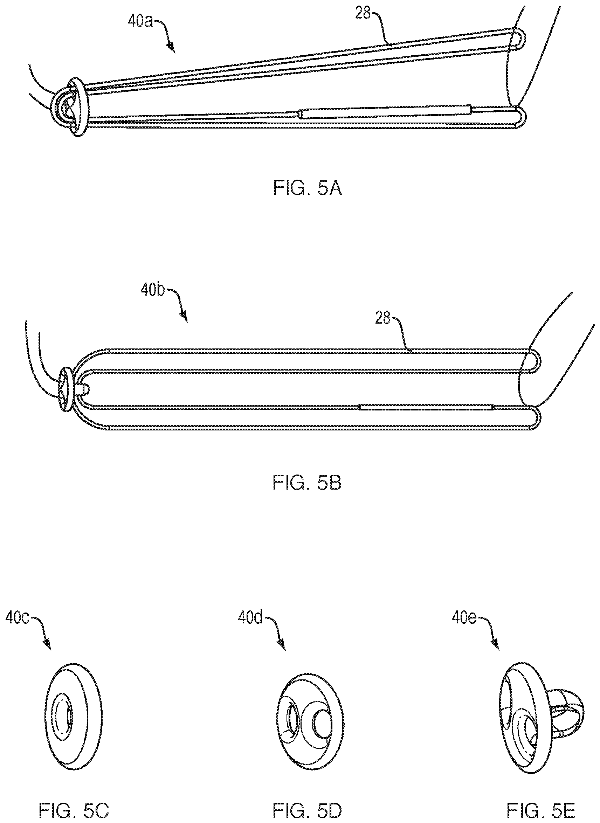

Examples of ancillary fasteners 40 are illustrated in FIGS. 5A-E. Ancillary fasteners 40a and 40b (FIGS. 5A and 5B) comprise a device with a tension suture 28 pre-attached. Ancillary fastener 40a comprises two through holes in the device for passage of a suture, and ancillary fastener 40b additionally comprises a short, open post attached to the device for passage of a suture. Ancillary fasteners 40c-e (FIGS. 5C-E) comprise a device with one through hole (40c), two through holes (40d) or two through holes and a short, open post (40e) for passage of a suture. Either of the fixation devices 10, 30 may be coupled with the tension suture 28 as described above to one of the ancillary fasteners 40 shown in FIGS. 5A-E to form an enhanced tissue fixation construct. For example, fixation device 10 may be coupled to either of the ancillary fasteners 40a, 40b, and fixation device 30 may be coupled to one of the ancillary fasteners 40c, 40d, 40e, to form an enhanced tissue fixation construct.

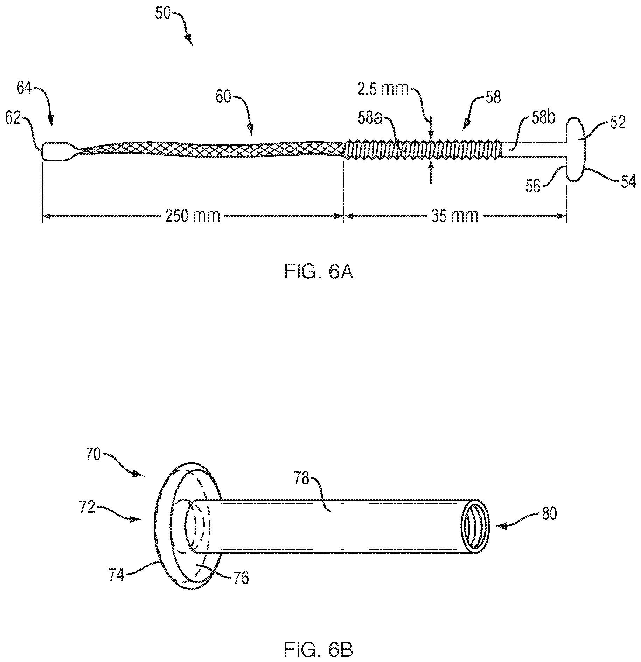

Turning now to FIG. 6A, another example of a fixation device 50 according to the present disclosure is shown. The fixation device 50 is substantially similar to the fixation devices 10, 30 except as described below. The fixation device 50 includes a generally circular body 52 having a first surface 54, which may be flat or convex, for facing away from bone, and an opposite concave (or bowl-shaped) second surface 56, for facing bone. The fixation device 50 further includes a generally cylindrical, solid post 58 fixedly coupled to a center of the body 52 and extending perpendicular to the body 52. An overall length of the post 58 is selected to extend through both of a bone graft and a portion of bone, such as a glenoid bone. For example, a length of the post 18 may be about 35 mm and a width of the post may be about 2.5 mm. The post 58 further comprises a threaded distal portion 58a and a non-threaded proximal portion 58b, the purpose of which will be described in more detail below. The fixation device 50 also includes a length of flexible cable 60, which may comprise braided suture, coupled to the distal end of the post 58. In examples, a length of the cable 60 may be about 250 mm. A flexible element 62, such as a suture, may be threaded through the cable 60 such that it forms a loop 64 at the distal end of the cable 60, as described in more detail below.

FIG. 6B illustrates an exemplary ancillary fastener 70 for use with the fixation device 50 of FIG. 6A during a graft fixation procedure. The fastener 70 is substantially similar to fixation device 10 except as described below. The fastener 70 comprises a generally circular body 72 having a first surface 74, which may be flat or convex, for facing away from bone, and an opposite concave (or bowl-shaped) second surface 76, for facing bone. The fastener 70 further includes a generally cylindrical, solid post 78 fixedly coupled to a center of the body 72 and extending perpendicular to the body 72. An overall length of the post 78 may be about 25 mm and a width of the post 78 may be about 3.5 mm. The fastener 70 further comprises a cannulation 80 extending through the body 72 and the post 78. At least a portion of the cannulation 80 is threaded to engage the threaded distal portion 58a of the fixation device 50, as further described below.

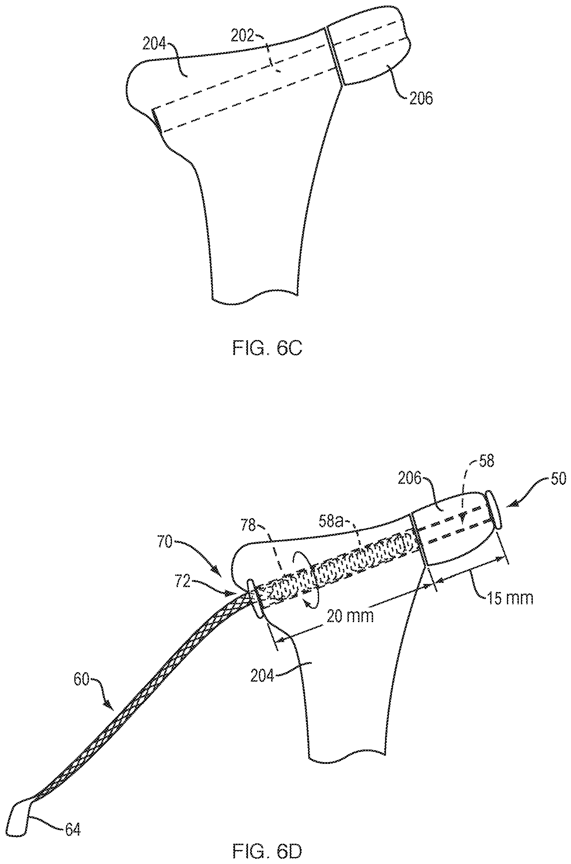

FIGS. 6C and 6D illustrate a method of tissue repair using the fixation device 50 and the ancillary fastener 70 as described above. Referring now to FIG. 6C, a passage 202 is drilled through the patient's glenoid 204 and a bone graft 206. A width of the passage 202 through the glenoid 204 may be about 3.8 mm and a width of the passage 202 through the bone block may be about 2.5 mm. As shown in FIG. 6D, the post 78 of the ancillary fastener 70 is inserted into the passage 202 in the glenoid 204 until the body 72 of the ancillary fastener 70 is positioned below the glenoid 204. The loop 64 and the cable 60 of the fixation device 50 are pulled through the bone graft 206 and the cannulation 80 (FIG. 6B) of the fastener 70 until the loop 64 and the cable 60 exit the passage 202 below the glenoid 204. The post 58 of the fixation device 50 is also inserted into the in the bone graft 206 and the cannulation 80 of the fastener 70. The fixation device 50 is then rotated such that the threaded distal end 58a of the post 58 engages the threads of the cannulation 80 of the fastener 70, securing the bone graft 206 against the glenoid 204. Once the bone graft 206 is secured, the cable 60 can be cut and the repair completed.

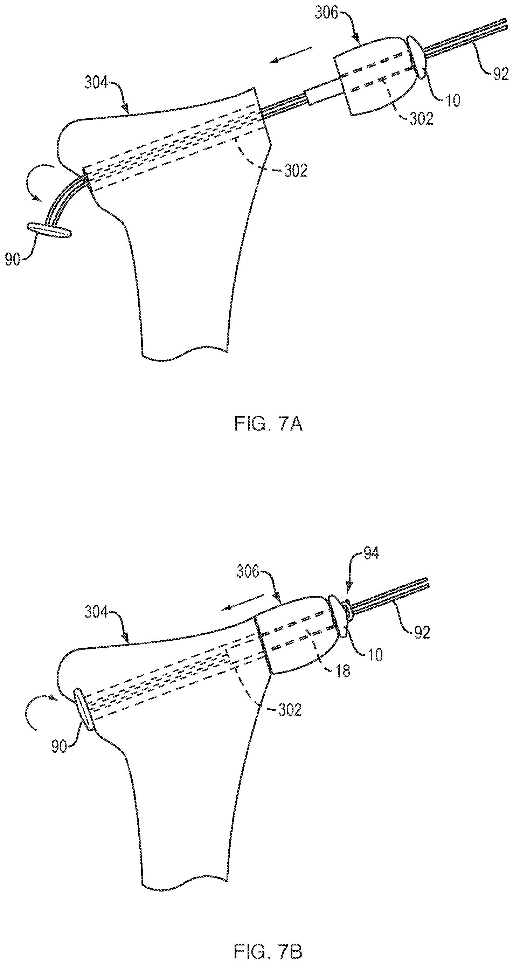

FIGS. 7A and 7B illustrate yet another method of tissue repair using, for example, the fixation device 10 of FIGS. 1A and 1B, and a rectangular fastener 90. As shown in FIG. 7A, a passage 302 is initially drilled through both of the patient's glenoid 304 and a bone graft 306. A tension suture 92 coupled to the fastener 90 is pulled through the passage 302 in the patient's glenoid 304 and the fixation device 10 until the ends of the tension suture 92 extend from the fixation device 10. A distance between the fastener 90 and the fixation device 10 can thus be adjusted by pulling on the ends of the tension suture 92. It should be noted that, while only one passage 302 is shown in FIGS. 7A and 7B, two passages 302 (and thus two fixation devices 10 and two fasteners 90) may be used in the repair. Pulling on the ends of the tension suture 92 causes the fastener 90 to flip from a position relatively parallel to the passage 302 (FIG. 7A) to a position relatively perpendicular to the passage 302 (FIG. 7B) such that the fastener 90 cannot enter the passage 302. The ends of the tension suture 92 are pulled until the fastener 90 is positioned below the glenoid 304. As the ends of the tension suture 92 are pulled, the fixation device 10 is reduced toward the glenoid 304 such that the post 18 is inserted at least partially into the passage 302 in the glenoid 304, forming a slip fit with the passage 302. Once the bone graft 306 is in the preferred position, a surgical knot 94 is tied in the tension suture 92 above the fixation device 10, fixing the bone graft 306 into place. The ends of the tension suture 92 may then be trimmed and the repair completed.

## Claims

1. A tissue fixation construct comprising:
a first fastener (50) comprising:
a generally flat, circular first body (52); and
a generally cylindrical, solid first post (58) fixedly coupled to a center of the first body (52) and extending perpendicular to the first body (52), the first post (58) comprising a threaded distal end (58a) and a non-threaded proximal end (58b), a length of the first post selected to extend through a bone graft (206) and a portion of bone;
a second fastener (70) comprising:
a generally flat, circular second body (72); and
a generally cylindrical, solid second post (78) fixedly coupled to a center of the second body (72) and extending perpendicular to the second body (72); and
a cannulation (80) extending through the second body (72) and the second post (78);
wherein at least a portion of the cannulation is threaded to engage the threaded distal end (58a) of the first post (58) of the first fastener (50), **characterized in that**
a length of flexible cable (60) is coupled to the distal end of the first post (58),
wherein the flexible cable (60) is configured to be pulled through the cannulation (80) of the second fastener (70).

2. The construct of claim 1, wherein a length of the first post (58) is about 35 mm.

3. The construct of claim 1,wherein the length of flexible cable (60) comprises a braided suture.

4. The construct of any one of the preceding claims, wherein a distal end of the length of flexible cable (60) comprises a loop (64).

## Patentansprüche

1. Gewebefixierungskonstrukt, umfassend:
ein erstes Befestigungselement (50), umfassend:
einen im Allgemeinen flachen, kreisförmigen ersten Körper (52); und
einen im Allgemeinen zylindrischen, festen ersten Pfosten (58), der fest mit einem Zentrum des ersten Körpers (52) gekoppelt ist und sich senkrecht zu dem ersten Körper (52) erstreckt, wobei der erste Pfosten (58) ein mit einem Gewinde versehenes distales Ende (58a) und ein gewindefreies proximales Ende (58b) umfasst, wobei eine Länge des ersten Pfostens dazu ausgewählt ist, sich durch ein Knochentransplantat (206) und einen Teil des Knochens zu erstrecken;
ein zweites Befestigungselement (70), umfassend:
einen im Allgemeinen flachen, kreisförmigen zweiten Körper (72); und
einen im Allgemeinen zylindrischen, festen zweiten Pfosten (78), der fest mit einem Zentrum des zweiten Körpers (72) gekoppelt ist und sich senkrecht zu dem zweiten Körper (72) erstreckt; und
eine Kanülierung (80), die sich durch den zweiten Körper (72) und den zweiten Pfosten (78) erstreckt;
wobei mindestens ein Teil der Kanülierung ein Gewinde hat, um das mit einem Gewinde versehene distale Ende (58a) des ersten Pfostens (58) des ersten Befestigungselements (50) in Eingriff zu nehmen, **dadurch gekennzeichnet, dass** eine Länge eines flexiblen Kabels (60) mit dem distalen Ende des ersten Pfostens (58) gekoppelt ist, wobei das flexible Kabel (60) dazu ausgelegt ist, durch die Kanülierung (80) des zweiten Befestigungsmittels (70) gezogen zu werden.

2. Konstrukt nach Anspruch 1, wobei eine Länge des ersten Pfostens (58) ungefähr 35 mm beträgt.

3. Konstrukt nach Anspruch 1, wobei die Länge des flexiblen Kabels (60) ein geflochtenes Nahtmaterial umfasst.

4. Konstrukt nach einem der vorhergehenden Ansprüche, wobei ein distales Ende der Länge des flexiblen Kabels (60) eine Schlaufe (64) umfasst.

## Revendications

1. Construction de fixation de tissu comprenant :
un premier élément de fixation (50) comprenant :
un premier corps (52) généralement plat et circulaire ; et
un premier montant (58) solide, généralement cylindrique, couplé de manière fixe à un centre du premier corps (52) et s'étendant perpendiculairement au premier corps (52), le premier montant (58) comprenant une extrémité distale filetée (58a) et une extrémité proximale non filetée (58b), une longueur du premier montant étant sélectionnée pour s'étendre à travers un greffon osseux (206) et une partie d'os ;
un second élément de fixation (70) comprenant :
un second corps (72) généralement plat et circulaire ; et
un second montant (78) solide, généralement cylindrique, couplé de manière fixe à un centre du second corps (72) et s'étendant perpendiculairement au second corps (72) ; et
une canulation (80) s'étendant à travers le second corps (72) et le second montant (78) ;
au moins une partie de la canulation étant filetée pour venir en prise avec l'extrémité distale filetée (58a) du premier montant (58) du premier élément de fixation (50), **caractérisée en ce qu'**une longueur de câble flexible (60) est couplée à l'extrémité distale du premier montant (58), le câble flexible (60) étant configuré pour être tiré à travers la canulation (80) du second élément de fixation (70).

2. Construction selon la revendication 1, une longueur du premier montant (58) étant d'environ 35 mm.

3. Construction selon la revendication 1, la longueur du câble flexible (60) comprenant une suture tressée.

4. Construction selon l'une quelconque des revendications précédentes, une extrémité distale de la longueur de câble flexible (60) comprenant une boucle (64).
